# EUROPEAN PATENT APPLICATION

(11) **EP 1 473 037 A1**
(43) Date of publication of application: **03.11.2004**
(21) Application number: 03076299.1
(22) Date of filing: 02.05.2003
(51) Int. Cl.: A61K 31/7056, A61P 31/14

(54) **Treatment of hPMV infections with Ribavirin**

(71) Applicant: Vironovative B.V., 3062 PA Rotterdam (NL)
(72) Inventor: Maertzdorf, Jeroen, 3513 SZ Utrecht (NL); Simon, James Henry Matthew, 3012 BM Rotterdam (NL)
(74) Representative: Prins, Adrianus Willem, Mr. Ir.

(57) **Abstract**

The invention relates to antiviral agents and compositions comprising such agents to treat and/or prevent respiratory diseases, in particular those caused by human metapneumovirus. Provided is a method for treating or preventing respiratory tract infections in a subject infected with a mammalian MPV, said method comprising administering a nucleoside analog, preferably Ribavirin or a derivative thereof, to said subject, and use of said nucleoside analog for the manufacture of a medicament for treating or preventing respiratory tract infections in a subject infected with a mammalian MPV.

## Description

The invention relates to the field of virology. More specifically, the invention relates to antiviral agents and compositions comprising such agents to treat and/or prevent respiratory diseases, in particular those caused by human metapneumovirus.

Human metapneumovirus (hMPV) is a respiratory viral pathogen that causes a spectrum of illnesses, ranging from asymptomatic infection to severe bronchiolitis. In 2001, van den Hoogen et *al.* (Nature Medicine 7, 719) described the identification of this new human viral pathogen from respiratory samples submitted for viral culture during the winter season. hMPV is a negative-sense nonsegmented RNA virus that has been categorized in the pneumovirus subfamily, family Paramyxoviridae, based on genomic sequence and gene constellation.

Little is known about the pathophysiology of hMPV infection, but hMPV appears to have a tropism for the respiratory epithelium. The respiratory viral pathogen hMPV causes a spectrum of illnesses, which range from asymptomatic infection to severe bronchiolitis. RSV, parainfluenza virus type 1, hMPV, and human parainfluenza virus type 3 (PIV3) are all known to cause clinical bronchiolitis. No medicine for the prevention or treatment of disease caused by MPV is available thus far.

The invention has now identified a nucleoside analog that has an inhibitory effect on hMPV replication and can thus be used to combat mammalian MPV infections. Provided is a method for reducing infection of a cultured cell by hMPV, said method comprising administering a nucleoside analog to said cell. Nucleoside analogs are (synthetic) chemical compounds created by modifying nucleosides, which are the natural building blocks of human and viral DNA and RNA. A nucleoside typically comprises a heterocyclic nitrogenous base, particularly a purine or pyrimidine, in N-glycosidic linkage with a sugar, particularly a pentose. Much antiviral and anticancer research has focused on nucleoside analogs, because viruses and cells use nucleosides to multiply. By mimicking the role of nucleosides in the cell division process, nucleoside analogs have been used to treat viruses and cancers by modifying the natural structure of DNA and RNA in a way that disrupts the viral and cellular replication machinery. Some nucleoside analogs have also been found to stimulate an antiviral immune response.

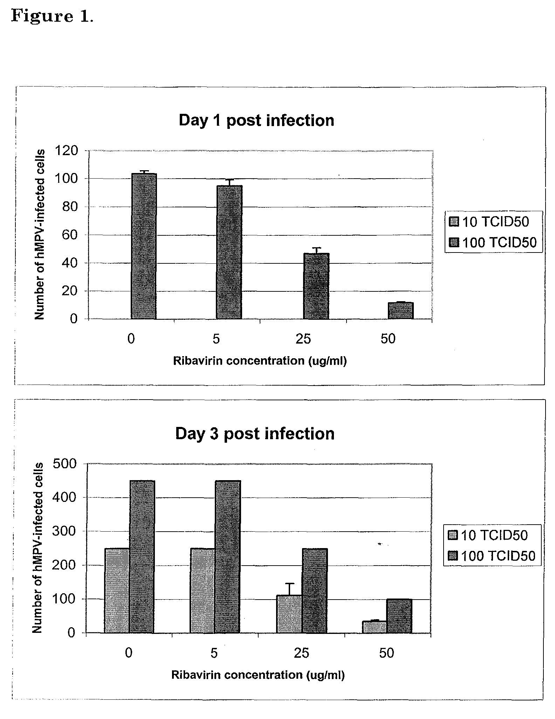

In a preferred embodiment of the invention, said nucleoside analog is a guanosine analog. More preferred, said guanosine analog is 1-(5-Deoxy-[beta]-D-ribo-furanosyl)-1,2,4-triazole-8-carboxamide, also known as Ribavirin or Virazole, or a derivative thereof. As is exemplified below (Fig.1), it was observed that treatment of cultured cells with Ribavirin prior to infection with human MPV significantly reduced the number of infected cells in a dose-dependent manner. Herewith, we have identified the nucleoside analog Ribavirin as a prophylactic agent against a mammalian MPV. What is more, the analog is also capable of reducing the replication rate of MPV when administered to cells post-infection. As is exemplified herein, following infection with hMPV, cells were treated after different time intervals (0-16 hours) with various concentrations (0-100 microgram/ml) of Ribavirin. Staining of the cells for the presence of hMPV revealed that Ribavirin at a concentration of 25 microgram/ml or higher greatly reduced the percentage of MPV-positive cells 3 days post-infection. Thus, in addition to the prophylactic effect against MPV, the nucleoside analog also has a therapeutic effect because it can decrease viral replication post-infection.

Ribavirin is currently indicated as an anti-viral drug for the treatment of chronic hepatitis C. Also, the anti-viral activity of Ribavirin was tested against RSV in vitro (Hruska 1980 Antimicrob. Agents Chemother. 17, 770) and in vivo (Hruska 1982 Antimicrob. Agents Chemother. 21, 125). Ribavirin has been used in a number of clinical trials to investigate its effectiveness in treating RSV infections, predominantly in infants and children with RSV infection and lower respiratory tract infection. Antiviral treatment with Ribavirin is currently the only approved antiviral prescription therapy for RSV. However, randomized trials comparing Ribavirin with placebo showed that Ribavirin does not always have positive effects.

Our finding that Ribavirin decreases replication of MPV is surprising, because Ribavirin does not work against all members of the *Paramyxoviridae* family. For example, Nichols *et al.* (Blood 2001 volume 98, 3:573-578) and Elizaga *et al.* (Clin Infect Dis 2001, 32 :413-418) reported that Ribavirin is not an effective agent against parainfluenza virus, which belongs to the same family as MPV and RSV. The study by Elizaga et al. involved treating 18 of 24 patients infected with PIV type 3 (PIV3) with a Ribavirin treatment starting only three days after the onset of symptoms. However, despite this early treatment with Ribavirin, no improvement in outcome was observed. In the study by Nichols *et al.* 31 of 55 patients with PIV3 pneumonia were treated with Ribavirin therapy within 48 hours of diagnosis. Also here, characteristics (viral shedding) of treated and untreated patients were similar and the 30-day mortality rate did not appear to be affected by the administration of Ribavirin. These data clearly indicate that Ribavirin is not active against all members of the paramyxovirus family, thereby underscoring the unexpected results shown in the present invention.

In a preferred embodiment, the invention provides a method for treating or preventing respiratory tract infections in a subject infected with a mammalian MPV, said method comprising administering a nucleoside analog to said subject, preferably Ribavirin or a derivative thereof. For example, a nucleoside analog can be used to treat a patient infected with human MPV (hMPV). The nucleoside analog can be administered to a subject in various ways, for instance orally, intranasally or intravenously. Typically, Ribavirin is administered to a subject using a small-particle aerosol generator. Also, an endotracheal tube can be used to administer a nucleoside analog to a subject. A typical treatment regimen is 1-500, more preferably 10-100, more preferably 10-30 and for example 20 mg/ml Ribavirin as the starting solution in the drug reservoir of a small-particle aerosol generator, with continuous aerosol administration for 12-18 hours per day for 3 to 7 days. Using a drug concentration of 20 mg/ml the average aerosol concentration for a 12 hour delivery period would be 190 micrograms/liter of air. However, the dosage regimen of the nucleoside analog and the concentration used may be varied according to clinical insights. Aerosolized Ribavirin should preferably not be administered in a mixture for combined aerosolization or simultaneously with other aerosolized medications

Furthermore, a method is provided for treating or preventing respiratory tract infections in a subject infected with a mammalian MPV and co-infected with one or more viruses from the Paramyxoviridae family, said method comprising administering a nucleoside analog, preferably Ribavirin or a derivative thereof, to said subject. A subject may be co-infected with one or more viruses from the Paramyxoviridae family belongs to the Pneumovirinae sub-family, such as Respiratory Syncitial Virus (RSV).

In another aspect, the invention provides a method for treating or preventing respiratory tract infections in a subject infected with mammalian MPV and co-infected with one or more other respiratory pathogens such as viruses, bacteria, yeast, fungi and mycoplasma, said method comprising administering a nucleoside analog to said subject. In a preferred embodiment, said nucleoside analog is Ribavirin. Said subject may for instance be co-infected with one or more other RNA viruses, for example with a member of the Coronavirus family such as a severe acute respiratory syndrome (SARS)-related Coronavirus.

A method of the invention is advantageously used to treat or prevent disease in a human subject, preferably a human subject considered at high risk for viral infections such as an infant subject of less than 5 years old, preferably less than 2 years old, an elderly subject, or an immunocompromised subject. In an immunocompromised subject, the immune system is functioning below normal. This makes someone more susceptible to viral, fungal, or bacterial infections. Those who can be considered to be immunocompromised include AIDS patients (or HIV positive), diabetics, transplant patients (on immunosuppressive drugs), and those who are receiving chemotherapy for cancer.

In a further embodiment, said human subject additionally suffers from a disease or condition other than a respiratory tract infection, for example cystic fibrosis, non-Hodgkin lymphoma, asthma, bone marrow transplantation or kidney transplantation. A method of the invention can also be used to treat or prevent respiratory tract infections in a subject infected with a mammalian MPV wherein said subject suffers from SARS, said method comprising administering a (guanosine) nucleoside analog, preferably Ribavirin, to said subject.

The invention provides the use of a nucleoside analog, preferably Ribavirin or a derivative thereof, for the manufacture of a medicament for treating or preventing respiratory tract infections in a subject infected with a mammalian MPV such as hMPV. Also provided herein is the use of a nucleoside analog for the manufacture of a medicament for treating or preventing respiratory tract infections in a subject infected with a mammalian MPV and co-infected with another virus, preferably one or more viruses from the Paramyxoviridae family, for example a virus that belongs to the Pneumovirinae sub-family such as Respiratory Syncitial Virus (RSV). It should be noted that the activity of Ribavirin is not restricted to viruses only. For example, it has been reported that certain micro-organisms (e.g. Pseudomonas spp.) are sensitive to Ribavirin. (Kruszewska et al., Acta Pol Pharm 2002, 59(6):436-9). In one embodiment of the invention, use of a nucleoside analog is provided for the manufacture of a medicament for treating or preventing respiratory tract infections in a subject infected with mammalian MPV and co-infected with one or more other respiratory pathogens such as viruses, bacteria, yeast, fungi and mycoplasma. Such a medicament may be used to treat or prevent a respiratory tract infection in a human subject, for instance an infant of less than 5 years old, preferably less than 2 years old. Also, elderly human subjects can be treated with a medicament comprising a nucleoside analog (preferably Ribavirin) of the invention. Furthermore, said nucleoside can be used for the manufacture of a medicament or pharmaceutical composition to treat a subject that additionally suffers from a disease or condition other than a respiratory tract infection, such as cystic fibrosis, non-Hodgkin lymphoma, asthma, bone marrow transplantion or kidney transplantation. In a further embodiment, use of a nucleoside analog, preferably Ribavirin, is provided for the manufacture of a medicament for treating or preventing a respiratory tract infection in a human subject suffering from SARS.

It has been shown that Ribavirin alone is not effective against hepatitis C virus infection in the long term. When Ribavirin is used in combination with the drug interferon, researchers have found that about twice as many people as those using Ribavirin alone show a long term clearance of detectable hepatitis C virus from the blood. Also, it was reported that high doses of Ribavirin to treat hepatitis C can be associated with several side effects, including leukopenia and haemolytic anemia. In order to reduce the occurrence or severity of side effects, co-administration of Ribavirin with interferon alpha-2B has been introduced for the treatment of hepatitis C (Reichard et *al.,* Lancet 1991;337:1058). In one aspect of the invention, a nucleoside analog is used for the manufacture of a medicament for treating or preventing respiratory tract infections caused by mammalian MPV, wherein said medicament further comprises a cytokine, preferably interferon, such as interferon alpha, beta or gamma. More preferred, a medicament or pharmaceutical composition of the invention comprises Ribavirin and interferon alpha-2B. Said interferon may be pegylated interferon. Pegylated interferon is produced when chemical substances called polyethylene glycol (PEG) are attached to interferon. The PEG attachment to interferon helps the interferon to act in a number of ways. It shields the interferon from the body, so that it slows the rate at which the immune system attacks and breaks down the interferon. In addition, the PEG-interferon molecule is larger. This means it is able to stay in the circulation for longer as it is less likely to leak out into other tissues and it is also filtered and removed by the kidneys at a slower rate.

### Example 1: Inhibitory effect of Ribavirin on HMPV replication

### Materials and methods

### Cell lines and Virus

Vero cells (African green monkey kidney cells) were maintained and passaged in Iscove's Modified Dulbecco's Medium (IMDM) containing L-glutamine (2mM), penicillin (100U/ml), streptomycin (100µg/ml) and 10% fetal calf serum (FCS) and incubated in a humidified atmosphere at 37°C. Virus infection medium contained bovine serum albumin (BSA;0.3%) and trypsin (2.5x10⁻⁴%) instead of FCS. Human Metapneumovirus stocks were prepared by inoculating Vero cells with hMPV, and harvesting 7-10 days post-infection, when there were visible cytopathic effects. The virus was stored at -70°C and titrated by end point dilution on Vero cells.

### Ribavirin as a prophylactic

One day prior to infection, the cells were seeded in 24 well plates at 30-40% confluency, and incubated overnight at 37°C. The next day, cells were pre-incubated with fresh medium containing 0, 5, 25 or 50 µg/ml of Ribavirin for 2 hours prior to virus infection. Following this preincubation, cells were infected with 10 or 100 TCID50 (tissue culture infectious doses) of hMPV, in the continuing presence of Ribavirin. After 2 hours, the virus inoculum was washed off and fresh medium containing Ribavirin was put onto the cells.

### Ribavirin as α therapeutic

Cells were seeded in 24 well plates as described above. The next day, cells were inoculated with 10 or 100 TCID50 of hMPV in the absence of Ribavirin. Two hours post-infection, the supernatant was aspirated and the cells were washed in phosphate-buffered saline (PBS) to remove virus inoculum. Fresh medium was added and the cells were incubated at 37 °C. At different time points (2, 4, 8 and 16 hours) after infection, Ribavirin was added to the cells to reach concentrations of 0, 5, 25 or 50 µg/ml.

### Analysis of Ribavirin effect

The plates were incubated at 37°C for 1 or 3 days. Medium was refreshed every second day and Ribavirin was maintained in the medium at all times at the appropriate concentration. After the indicated incubation period, medium was removed, the cells were washed with PBS and cells were fixed with 80% acetone. Staining for immune fluorescence analysis was performed by incubating infected cells with guinea pig anti-hMPV serum in PBS for 1 hour, followed by a FITC-labeled rabbit anti-guinea pig polyclonal antibody preparation (DAKO) in PBS for 1 hour. Background staining was finally performed with eriochrome black before analysis under an immune fluorescence microscope. Infected cells were counted in 5 fields under high power magnification (320x). Results are presented as the total number of infected cells counted in these 5 fields

### Results

### Ribavirin as a prophylactic

The results from the immune fluorescence analysis are presented in Figure 1. At day 1 post- infection, few infected cells were visible in the wells infected with 10 TCID50 of hMPV. In contrast, in wells infected with 100 TCID50 of virus, there were sufficient infected cells to count. Figure 1 demonstrates that the addition of Ribavirin shows a clear dose-dependent effect on virus replication, with a reduction in number of infected cells of approximately 50% in the presence of 25 µg/ml Ribavirin and of approximately 95% with 50 µg/ml of Ribavirin.

The same effect was seen at day 3 after infection for cells infected with both 10 or 100 TCID50 of virus. This is represented in figure 1 as 500 infected cells as each field under the microscope represents approximately 100 cells. The third time point was at day 6 after infection, at which time almost all cells were infected (data not shown).

### Ribavirin as α therapeutic

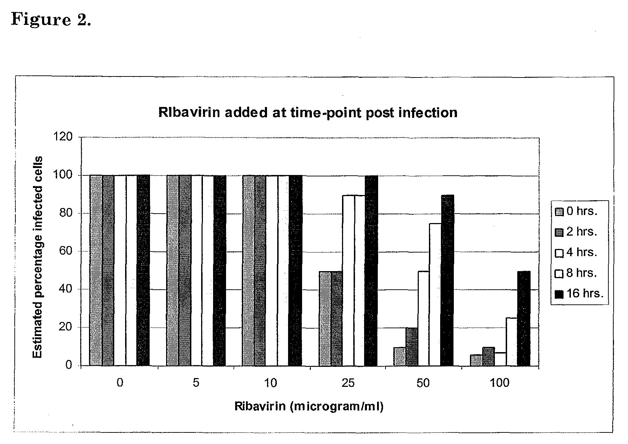

The results depicted in Figure 2 demonstrate that Ribavirin inhibits replication of hMPV when added post-infection. Furthermore, the data indicate that the higher the concentration of Ribavirin and the earlier post-infection that Ribavirin is added, the more effective it is at inhibiting hMPV replication. For example, Ribavirin at 100µg/ml is effective at reducing infection by 95% when added at 0, 2, or 4 hours, by 80% when added 8hrs post-infection and by 50% when added 16hrs post-infection. In contrast, administration of Ribavirin in a dose of 25 µg/ml inhibits viral replication by 50% when added at 0 or 2hrs post-infection, by 10% when added 4 or 8 hrs post-infection and insignificantly when added after 16 hrs.

### Conclusions

Our results show that Ribavirin inhibits hMPV growth in cell culture both when added prior to or after virus infection. A decrease in the number of infected cells of up to 95% was measured when 50 µg/ml of Ribavirin was added to cell cultures 2 hrs prior to or at the same time as virus infection. In order to see a significant effect 8 or 16 hrs post-infection, the concentration of Ribavirin has to be increased to 100µg/ml.

### LEGENDS

### Figure 1.

Number of hMPV infected cells as determined by immune fluorescence. Five fields were counted under the microscope at a high power magnification (320x).

### Figure 2.

Percentage of cells infected with hMPV. Cells were infected with 30 TCID50 of hMPV and after 0,2, 4, 8 and 16 hours, post-infection, 0, 5, 10, 25, 50 or 100 microgram of Ribavirin was added. Three days post-infection, cells were fixed and stained with a hMPV-specific polyclonal antibody, and the number of hMPV positive and negative cells were counted in 5 fields.

## Claims

1. Use of a nucleoside analog for the manufacture of a medicament for treating or preventing respiratory tract infections in a subject infected with a mammalian MPV.

2. Use according to claim 1, wherein said nucleoside analog comprises Ribavirin or a derivative thereof.

3. Use of a nucleoside analog, preferably Ribavirin or a derivative thereof, for the manufacture of a medicament for treating or preventing respiratory tract infections in a subject infected with a mammalian MPV and co-infected with one or more viruses from the Paramyxoviridae family.

4. Use of a nucleoside analog, preferably Ribavirin or a derivative thereof, for the manufacture of a medicament for treating or preventing respiratory tract infections in a subject infected with mammalian MPV and co-infected with one or more other respiratory pathogen See e-mail.

5. Use according to any one of claims 1-4, wherein said subject is a human subject less than 5 years old, preferably less than 2 years old.

6. Use according to any one of claims 1-5, wherein said subject is a human subject suffering from SARS.

7. Use according to any one of claims 1-6, wherein said medicament further comprises interferon, preferably interferon alpha-2B.

8. A method for treating or preventing respiratory tract infections in a subject infected with a mammalian MPV, said method comprising administering a nucleoside analog, preferably Ribavirin or a derivative thereof, to said subject.

9. A method according to claim 8, wherein said mammalian MPV is hMPV.

10. A method for treating or preventing respiratory tract infections in a subject infected with a mammalian MPV and co-infected with one or more viruses from the Paramyxoviridae family, said method comprising administering a nucleoside analog, preferably Ribavirin or a derivative thereof, to said subject.

11. A method according to claim 10, wherein said subject is co-infected with a virus that belongs to the Pneumovirinae sub-family, preferably Respiratory Syncitial Virus (RSV).

12. A method for treating or preventing respiratory tract infections in a subject infected with mammalian MPV and co-infected with one or more other respiratory pathogens, said method comprising administering a nucleoside analog, preferably Ribavirin or a derivative thereof, to said subject.

13. A method according to claim 12, wherein said subject is co-infected with one or more other RNA viruses, preferably with a member of the Coronavirus family, more preferred with SARS-related Coronavirus.

14. A method according to any of claims 8-13, wherein the subject is human.

15. A method according to claim 14, wherein the subject is less than 5 years old, preferably less than 2 years old.

16. A method according to claim 14, wherein the subject is an elderly.

17. A method according to any one of claims 14-16, wherein the subject additionally suffers from a disease or condition other than a respiratory tract infection, preferably a disease or condition is selected from a group essentially consisting of cystic fibrosis, non-Hodgkin lymphoma, asthma, bone marrow transplantation and kidney transplantation.

18. A method according to any one of claims 14-16, wherein the subject is immunocompromised.

19. A method according to any one of claims 14-16, wherein the subject suffers from SARS.
